# EUROPÄISCHE PATENTSCHRIFT

(11) **EP 2 279 701 B1**
(45) Veröffentlichungstag und Bekanntmachung des Hinweises auf die Patenterteilung: **02.09.2015**
(21) Anmeldenummer: 10171031.7
(22) Anmeldetag: 28.07.2010
(51) Int. Cl.: A61B 17/08, A61B 19/00

(54) **Vorrichtung zum Dehnen von Gewebebereichen**
Device for stretching areas of tissue
Dispositif d'étirement de zones tissulaires

(30) Priorität: 28.07.2009 DE 102009036165
(43) Veröffentlichungstag der Anmeldung: 02.02.2011
(73) Patentinhaber: Karl Storz GmbH & Co. KG, 78532 Tuttlingen (DE)
(72) Erfinder: Möhrle, Matthias Dr., 72070 Tübingen (DE)
(74) Vertreter: Witte, Weller & Partner Patentanwälte mbB

(56) Entgegenhaltungen:
- WO-A1-99/35974
- US-A- 4 535 772
- US-A- 5 127 412
- US-A1- 2004 254 609
- US-A1- 2007 255 315

## Beschreibung

Die Erfindung betrifft eine Vorrichtung zum Dehnen von Gewebebereichen, mit zumindest einem ersten plattenförmigen Körper, und mit zumindest einem zweiten plattenförmigen Körper, die gegenüberliegend am Gewebe anordenbar sind, mit zumindest einem Zugfaden, der die beiden plattenförmigen Körper untereinander verbindet, ferner mit zumindest einer Spannvorrichtung auf zumindest einem der beiden plattenförmigen Körper zum Beaufschlagen des zumindest einen Zugfaden mit einer Zugkraft, wodurch die beiden plattenförmigen Körper aufeinander zu bewegbar sind, und mit einer Fadenführung für die Zugelemente, über die die beiden plattenförmigen Körper zugkraftschlüssig untereinander verbindbar sind.

Solch eine Vorrichtung ist aus der WO 99/35974 A1 bekannt.

Die dort gezeigte Vorrichtung dient dazu Wunden zu schließen. Dazu weist sie zwei plattenförmige Verstärkungselemente auf, die auf der Haut aufgebracht werden und mit widerhakenförmigen Elementen im oberen Bereich der Haut gehalten werden. Die beiderseits der Wunde angebrachten Verstärkungselemente werden dann über ein Zugfaden, vorzugsweise ein Zugband, zunächst unterwärts durch die Haut und dann auf der Oberseite über die Wunde hinweg verbunden. Um dann eine Spannung in der erzeugten Zugbandschleife zu erzeugen, ist auf dem einen Verstärkungselement eine Spannvorrichtung vorgesehen. Diese Spannvorrichtung gestattet es das Zugfaden nach und nach zu spannen, in dem dieses Band auf der der Wunde zugewandten Seite in die Spannvorrichtung hineingeführt wird und dann gehalten durch eine einrastende Trommel auf der gegenüberliegenden Seite wieder herausgeführt wird.

Eine ähnliche Vorrichtung ist aus der US 4,535,772 bekannt.

Bei dieser Vorrichtung werden vorzugsweise zwei Reihen mit plattenförmigen Elementen an einer Wunde angeordnet und mit nadelartigen Pins an dieser Wundenkante gehalten. Zu dem Wundenrand hin, weisen die Vorrichtungen jeweils beinförmige Abschnitte auf, die nach oben herausstehen und so über eine Spannvorrichtung miteinander verbunden werden können. Diese Spannvorrichtungen können zum einen aus Klammern bestehen, oder aber auch Bänder mit Rasten und dazugehörige Aufnahmen aufweisen, um ein Aufeinanderzubewegen der Wundenränder durch allmähliches Nachspannen zu bewirken.

Die Dehnung von Gewebe, insbesondere von Hautgewebe, ist ein in der Chirurgie, insbesondere in der plastischen Chirurgie, häufig durchzuführender Vorgang.

Neben einer Hautdehnung findet ein Dehnen von Bindegewebe, Sehnen, Muskeln und auch von Gefäßen statt.

In der Dermatochirurgie, insbesondere in der plastisch-rekonstruktiven Chirurgie, ist ein Dehnen der Haut beidseits von Läsionen vor dem Entfernen der Läsion erforderlich.

Beim Exzidieren oder Entfernen von Läsionen handelt es sich unter anderem um die Entfernung von Hauttumoren, von angeborenen Muttermalen oder auch von großflächigen Narben.

Ist eine solche Läsion relativ großflächig, kann sie nicht durch einen einzigen Eingriff entfernt werden, denn die Hautbereiche um die Läsion müssen stark gedehnt werden, wobei diese Dehnung eine Rückstellkraft erzeugt, welche die Wundheilung stört oder die dazu neigt, die meist vernähte Operationsstelle wieder aufzureißen. Daher kommen bisher sogenannte serielle Exzisionen zum Einsatz. Das heißt, es werden zunächst nur Teile einer Läsion entfernt. Es wird danach abgewartet, bis sich die umgebende Haut nachgedehnt hat, was in der Regel Monate dauert, um dann einen weiteren Teil der Läsion und auch die Narbe der vorausgehenden Operation zu entfernen.

Dieser Vorgang ist nicht nur sehr aufwändig, sondern ist für den Patienten mit entsprechenden wiederholten Belastungen, Risiken, Kosten und beispielsweise Arbeitsunfähigkeiten verbunden.

Es wurden sogenannte Gewebsexpander entwickelt, die unter die umgebende Haut implantiert werden. Nach der Wundheilung wird der Gewebsexpander transkutan mit Flüssigkeit sukzessive gefüllt. Der so entstehende "Wasserballon" dehnt die umgebende Haut auf. Nach einigen Wochen kann der Expander entfernt werden. Dabei wird die Läsion exzidiert und der Defekt wird mit der durch Dehnung gewonnenen Haut gedeckt.

Derartige Gewebsexpander sind sehr teuer und nur einmal verwendbar. Das Verfahren besitzt ein hohes Infektionsrisiko, denn es ist ein Fremdkörper unter die Haut zu implantieren, der regelmäßig punktiert und nachgefüllt werden muss. Dabei sind häufige Vorstellungen bei hochspezialisierten Ärzten zur Operation und zum Auffüllen der Gewebsexpander notwendig. Der aufgefüllte Ballon ist deutlich sichtbar und schränkt über Wochen das private und berufliche Leben des Patienten ein.

Aus der DE 103 49 953 B4 ist eine Vorrichtung zum Fixieren und Spannen zumindest eines Zugfadens für das Anlegen einer Neovagina bekannt. Diese Vorrichtung weist einen plattenförmigen Grundkörper auf, an dem zumindest ein Fixierelement zum Fixieren des zumindest einen Zugfadens unter Spannung angeordnet ist, wobei dem Fixierelement eine Feder zugeordnet ist, deren erstes Ende am Grundkörper festgelegt ist. Das Fixierelement ist zum Spannen des zumindest einen Zugfadens um eine Drehachse drehbar, wobei eine Feststelleinrichtung für das Fixierelement vorhanden ist, mittels der das Fixierelement zumindest in einer Drehrichtung, die der Drehrichtung zum Spannen des zumindest einen Zugfadens entgegengerichtet ist, feststellbar ist. Das freie Ende des Zugfadens oder gegebenenfalls mehrerer Zugfäden ist mit einer Kunststoffolive oder einem sogenannten Steckphantom verbunden. Diese üben über die Zugkraft einen kontinuierlichen Druck auf das Vaginalgrübchen aus, wodurch innerhalb von einigen Tagen eine Neovagina ausgebildet wird.

Es ist Aufgabe der vorliegenden Erfindung, unter Einsatz der minimalinvasiven Operationstechnik eine rasche Gewebsdehnung innerhalb kurzer Zeit zu erzielen. Erfindungsgemäß wird die Aufgabe dadurch gelöst, dass die Fadenführung auf dem plattenförmigen Körper, der dem mit der Spannvorrichtung gegenüberliegt, zumindest zwei seitlich voneinander beabstandete Widerlager aufweist. Diese Maßnahmen haben nunmehr mehrere Vorteile.

Die beiden plattenförmigen Körper werden gegenüberliegend an das Gewebe angeordnet, beispielsweise beidseits einer zu exzidierenden Läsion auf der Haut. Das heißt, sie müssen nicht in oder unter das Gewebe implantiert werden.

Die beiden plattenförmigen Körper sind so ausgestaltet, dass sie über den Zugfaden untereinander verbindbar sind.

Die Fadenführung kann vorteilhafterweise so erfolgen, dass mit dem Faden die jeweiligen plattenförmigen Körper an dem Gewebe fixiert werden, und dass gleichzeitig über den Faden die plattenförmigen Körper miteinander verbunden werden. Dies kann durch einen einfachen Vorgang mit einer entsprechenden Ahle durchgeführt werden, d.h. der Faden kann an einem Ende an dem ersten plattenförmigen Körper fixiert werden und er wird durch das Gewebe zu dem gegenüberliegenden zweiten plattenförmigen Körper geführt. Das freie Ende des Fadens wird mit der Spannvorrichtung verbunden, über die eine Zugkraft auf den Faden ausgeübt wird. Diese Zugkraft verursacht, dass sich die beiden plattenförmigen Körper aufeinander zu bewegen. Dadurch wird das zwischen den gegenüberliegenden Plattenkanten gelegene Gewebe gestaucht, der in Bewegungsrichtung der beiden plattenförmigen Körper gesehen, "hinter" den plattenförmigen Körpern gelegene Gewebebereich wird dabei gedehnt.

Die Oberfläche und auch die Ausformung der plattenförmigen Körper kann so gewählt werden, dass diese schon dadurch in zugkraftübertragenden Eingriff mit dem Gewebe treten. Dazu gehören Vorsprünge, Noppen, Riffelungen etc. Dann kann im Prinzip ein Fixieren der plattenförmigen Körper am Gewebe durch den Zugfaden entfallen.

Durch eine Fixierung der plattenförmigen Körper an dem Gewebe sind die plattenförmigen Körper fest an dem Gewebe gehalten, so dass der Patient sich frei bewegen kann. Da der Faden von dem einen plattenförmigen Körper, an dem er befestigt ist, durch das Gewebe zu einem anderen plattenförmigen Körper geführt ist, und am anderen Ende mit der Spannvorrichtung verbunden ist, kann über diese Spannvorrichtung eine Zugkraft auf den Faden ausgeübt werden, die verursacht, dass die beiden plattenförmigen Körper aufeinander zu bewegt werden.

Die Steuerung und die Beobachtung dieser Verschiebevorgänge können von der Außenseite her einfach durchgeführt werden, da alle Bauteile auf dem Gewebe liegen, lediglich die Verbindung über den Faden erfolgt durch das Gewebe hindurch.

Das hat zum einen operationstechnisch den erheblichen Vorteil, dass die Fadensetzung ein sehr einfach durchzuführender Vorgang ist, der auch ein äußerst geringes Infektionsrisiko birgt. Das Setzen der plattenförmigen Körper ist somit beispielsweise durch einen Arzt im Rahmen einer ambulanten Behandlung innerhalb eines kurzen Zeitraumes durchzuführen.

Der Spannvorgang des Fadens und dessen Steuerung dahingehend, dass über einige Tage nach und nach die beiden plattenförmigen Körper aufeinander zu bewegt werden, kann von dem Patienten auch selbst durchgeführt werden.

Dazu erhält er von dem behandelnden Arzt einen bestimmten Plan, wie oft und wie viel er die Spannvorrichtung betätigt, was beispielsweise einfach dadurch geschehen kann, dass die Spannvorrichtung ein Stellrad aufweist, um das der Faden aufgewickelt wird.

Wird beispielsweise das Grundprinzip der eingangs genannten DE 103 49 953 A1 bezüglich der Spannvorrichtung herangezogen, so ist dort ein Rastmechanismus vorhanden, über den das Spannrad jeweils über eine gewisse Anzahl an Rasten verdreht werden kann, wodurch dann entsprechend der Faden gespannt wird, mit der Folge, dass in der vorliegenden Erfindung die beiden plattenförmigen Körper etwas aufeinander zu bewegt werden. Es ist also möglich, dem Patienten einen Plan zu geben, in welchen Zeitintervallen und in welchen Schrittgrößen er die beiden plattenförmigen Körper aufeinander zu bewegt, bis die entsprechende Hautdehnung bzw. Quetschung im Bereich der Läsion erfolgt ist, was innerhalb weniger Tage erfolgen kann.

Erst dann muss sich der Patient wieder zu einer Sitzung begeben, in der das Gewebe entnommen, die Wunde verschlossen und die Vorrichtung wieder entfernt wird.

Liegen die plattenförmigen Körper beidseits einer Läsion auf der Haut, kann die Läsion (z.B. Narbe, Muttermal) entfernt werden.

Der zwischen den plattenförmigen Körpern gestauchte Gewebebereich kann auch zur Gewebsgewinnung herangezogen werden, beispielsweise zum Brustaufbau nach einer Ablatio Mammae.

Es kann Gewebe einerseits gedehnt werden und andererseits zwischen den plattenförmigen Körpern Oberfläche und Volumen an Gewebe für Rekonstruktionen gewonnen werden.

Der Begriff plattenförmiger Körper ist in dem Sinne zu verstehen, dass ein flächiger Anlagebereich zum Anlegen und ggf. zum Fixieren am Gewebe vorhanden ist und dass die Körper beim Spannen des zumindest einen Zugfadens sich aufeinander zu bewegen, dazwischen Gewebe aufstauen und in Zugrichtung bzw. Bewegungsrichtung "dahinter" das Gewebe dehnen.

Insgesamt gesehen sind somit der Dehnungsvorgang und das Entnehmen des Gewebes durch wenige Eingriffe mit einem geringen Infektionsrisiko für den Patienten sehr einfach und wenig beeinträchtigend durchzuführen.

Die plattenförmigen Körper sind mehrfach verwendbar, denn solche Körper sind einfach zu reinigen, zu desinfizieren und zu sterilisieren. Die Form, die Größe, die Dicke und die Materialien der plattenförmigen Körper können jeweils so gewählt werden, dass sie für den entsprechenden Einsatzbereich am Gewebe am geeignetsten und für den Patienten verträglich sind.

Ist eine Läsion an einem großflächigen ebenen Hautbereich, beispielsweise im Brust- oder Rückbereich, vorhanden, können die plattenförmigen Körper eben und relativ groß ausgebildet werden.

Ist die Läsion an Hautbereichen mit starken Krümmungen, wie beispielsweise an Arm und Beinen oder an sehr empfindlichen Stellen, beispielsweise im Bereich des Kopfes vorhanden, können dann entsprechend kleine, leichte und der Krümmung angepasste plattenförmige Körper vorgesehen werden.

Das Grundprinzip bleibt immer gleich, die plattenförmigen Körper können an das Gewebe angebracht, ggf. fixiert und untereinander verbunden werden.

Die beiden plattenförmigen Körper müssen am Gewebe verankert werden, damit der Zugkraft, die auf den Faden einwirkt, ein Widerlager entgegengesetzt wird, das für die Umsetzung der Zugkraft in die aufeinander zu gerichtete Bewegung sorgt. Die Fadenführung kann so sein, dass der zumindest eine Zugfaden nicht nur die plattenförmigen Körper untereinander verbindet, sondern diese auch zugleich am Gewebe fixiert. Die Verankerung der plattenförmigen Körper kann auch durch Klammern erfolgen, so dass der Zugfaden nur die beiden plattenförmigen Körper miteinander verbindet.

Werden die plattenförmigen Körper beispielsweise beidseits einer Läsion an die Haut angebracht und subkutan untereinander verbunden, kann durch ein regelmäßiges Betätigen der Spannvorrichtung innerhalb weniger Tage schon eine ausreichende Hautdehnung erzielt werden. Die Konstruktion erlaubt eine sehr flache Bauweise, so dass beispielsweise die Vorrichtung, wenn sie an Körperstellen angelegt wird, die üblicherweise durch Bekleidung abgedeckt ist, von der Außenseite kaum ersichtlich ist. Dies trägt erheblich zu der sogenannten Patientencompliance bei und somit auch zum Erfolg einer einfachen und unkomplizierten Exzision der Läsion. Die zwischen den aufeinander zu bewegten Platten gestauchten Hautbereiche im Bereich der Läsion erleichtern dem Arzt auch das Entfernen dieser Hautbereiche, da diese durch das Stauchen schon von einem flächigen Grund in eine mehr linienförmige Struktur zusammengeführt sind. Somit wird auch dadurch der eigentliche Eingriff erleichtert.

Die Maßnahme, dass die Fadenführung auf dem plattenförmigen Körper, der dem mit der Spannvorrichtung gegenüberliegt, zumindest zwei seitlich voneinander beabstandete Widerlager aufweist, hat den Vorteil, dass die Zugkraft auf diesem plattenförmigen Körper nicht nur punktuell einwirkt, was beim Verschieben dieses plattenförmigen Körpers nach und nach zu einem Drehen führen könnte, sondern durch die seitlich voneinander beabstandeten Widerlager wird eine Zugkraft ausgeübt, die dazu führt, dass der plattenförmige Körper in einer mehr geradlinigen Bewegung auf den gegenüberliegenden plattenförmigen Körper zu bewegt wird.

Dadurch ist eine sichere geradlinige Steuerung der Bewegung möglich, so dass nicht periodisch kontrolliert werden muss, ob der plattenförmige Körper auch die gewünschte Bewegungsbahn durchläuft. Diese Mehrpunktanlage über die mehreren Widerlager hat auch den Vorteil, dass die Zugkräfte gleichmäßiger über den plattenförmigen Körper und den Faden in das Gewebe übertragen werden können, so dass eine möglichst wenig traumatische Gewebedehnung erfolgt.

In einer weiteren Ausgestaltung der Erfindung weist die Fadenführung an dem plattenförmigen Körper, der eine Spannvorrichtung trägt, zumindest eine Führungsstelle auf, über die der Faden der Spannvorrichtung zuführbar ist und dort an einem ersten Ende aufwickelbar ist.

Diese Maßnahme hat den Vorteil, dass dadurch die Anordnung der Spannvorrichtung auf dem entsprechenden plattenförmigen Körper als solche frei wählbar ist, z.B. wo diese am günstigsten ist, denn durch die Führungsstelle wird der Faden der Spannvorrichtung jeweils gezielt zugeführt.

In einer weiteren Ausgestaltung der Erfindung weist die Vorrichtung an dem plattenförmigen Körper, der eine Spannvorrichtung trägt, auch eine Fixierstelle für das andere Ende des Fadens auf.

Diese Maßnahme hat den Vorteil, dass an diesem einen plattenförmigen Körper beide Enden des Zugfadens verankert sind, also einmal an der Fixierstelle und zum anderen an der Spannvorrichtung. Dies erleichtert die Handhabung dahingehend, dass der Faden beispielsweise an einem Ende an diesem plattenförmigen Körper fixiert wird, dann durch das Gewebe, z.B. subkutan zum gegenüberliegenden als Gegenstück wirkenden plattenförmigen Körper geführt und von diesem dann wieder zurück zur Spannvorrichtung geführt wird.

In Zusammenwirken mit einem Gegenstück mit entsprechend mehreren Verankerungsstellen ist dann eine besonders gezielte und sichere Führung der beiden plattenförmigen Körper bei der aufeinander zu gerichteten Bewegung möglich.

In einer weiteren Ausgestaltung der Erfindung liegen dazu die Widerlager auf dem einen plattenförmigen Körper und die Führungsstelle und die Fixierstelle am anderen plattenförmigen Körper an den Ecken eines Viereckes.

Diese Ausgestaltung hat den Vorteil, dass beim Spannen des Fadens die gegenüberliegenden plattenförmigen Körper exakt gerichtet aufeinander zu bewegt werden. So können beispielsweise die vier Eckpunkte an den Ecken eines mehr oder weniger geometrisch exakten Rechteckes liegen, so dass die beiden plattenförmigen Körper exakt geradlinig aufeinander zu bewegt werden. Bei der Entfernung eines Muttermales, das meist eine runde Kontur aufweist, können die beiden plattenförmigen Körper bei dieser Ausgestaltung diametral gegenüberliegend an den Enden des Muttermals angelegt, an der Haut fixiert und untereinander verbunden werden. Nach dem Dehnen der Haut ist das Muttermal zwischen den beiden plattenförmigen Körpern entsprechend gestaucht oder gequetscht, wobei die Erstreckung entlang eines Durchmessers erfolgt, der in etwa um 90° versetzt zu dem Durchmesser ist, der in der aufeinander zu gerichteten Bewegungsrichtung liegt.

In einer weiteren Ausgestaltung der Erfindung sind die Widerlager, die Fixierstelle und die Führungsstelle als Öffnungen in den plattenförmigen Körpern ausgebildet, über die der zumindest eine Zugfaden durch die Plattenebene quer hindurchführbar ist.

Diese Maßnahme hat den Vorteil, dass solche Öffnungen in solchen plattenförmigen Körpern einfach herzustellen sind und auch gut reinigbar, desinfizierbar und sterilisierbar sind. Für die Handhabung ist das ebenfalls sehr einfach, der Arzt sticht beispielsweise von einer Öffnung auf einem plattenförmigen Körper ausgehend mit einer Ahle, an der der Faden befestigt ist, diesen in das Gewebe, z.B. in die Haut ein, führt den Faden subkutan bis zu der entsprechenden gegenüberliegenden Stelle und sticht die Haut wieder zur Außenseite durch.

Er kann dann auf die aus der Haut austretende Spitze den zweiten plattenförmigen Körper auffädeln, indem er die Spitze der Ahle samt dem Faden oder dem Faden allein durch diese Öffnung hindurchführt. Diesen Vorgang wiederholt er dann, indem er den Faden wieder zu dem anderen plattenförmigen Körper zurückführt und dann durch die entsprechende Öffnung hindurch zu der Spannvorrichtung führt und dann dieses Ende dort auffädelt.

Das heißt, dieser Vorgang der Festlegung der plattenförmigen Körper z.B. an der Haut und die Verbindung untereinander sind sehr einfach durchzuführen.

In einer weiteren Ausgestaltung der Erfindung sind auf einer Seite mehr als ein plattenförmiger Körper vorhanden, die jeweils mit einem gegenüberliegend angeordneten plattenförmigen Körper über jeweils einen Zugfaden verbunden sind.

Ist z.B. eine Läsion relativ groß oder entsprechend unregelmäßig oder gekrümmt ausgeformt, kann nicht nur jeweils ein plattenförmiger Körper beidseits der Läsion angelegt werden, sondern es können mehrere plattenförmige Körper angelegt werden, die aber jeweils an der Haut fixiert und subkutan miteinander verbunden werden müssen.

Damit kann sehr flexibel auch auf solche Läsionen eingegangen werden und entsprechende Hautdehnungen bewerkstelligt werden.

In einer weiteren Ausgestaltung der Erfindung weist ein plattenförmiger Körper eine Spannvorrichtung auf und dieser plattenförmige Körper ist mit mehreren plattenförmigen Körpern auf einer gegenüberliegenden Seite über jeweils einen Zugfaden verbunden, und die Zugfäden der mehreren plattenförmigen Körper werden durch die Fadenführung zu einer einzigen Spannvorrichtung geführt und sind von dieser einzigen Spannvorrichtung gleichzeitig spannbar.

Diese Maßnahme hat den Vorteil, dass über eine einzige Spannvorrichtung, der die mehreren Fäden von den mehreren gegenüberliegenden plattenförmigen Körpern zugeführt werden, alle Fäden gleichzeitig spannbar sind. Dies erleichtert die Handhabung insbesondere, wenn diese von dem Patienten selbständig durchgeführt wird.

In einer weiteren Ausgestaltung der Erfindung sind zwei gegenüberliegend gelegene plattenförmige Körper über jeweils zwei separate Zugfäden verbunden.

Diese Maßnahme hat den Vorteil, dass über die separaten Zugfäden die Möglichkeit eröffnet ist, an einem Zugfaden stärker zu ziehen als am anderen, so dass es möglich ist, die Ausrichtung der plattenförmigen Körper untereinander während der Gewebsdehnung zu verändern. Dies kann erstrebenswert sein, wenn an einem Endbereich eines solchen plattenförmigen Körpers mehr Gewebebereiche zur Dehnung zur Verfügung stehen als beispielsweise an einem anderen Endbereich eines solchen plattenförmigen Körpers.

In einer weiteren Ausgestaltung der Erfindung ist jedem separaten Zugfaden eine separate Spannvorrichtung zugeordnet.

Diese Maßnahme hat den Vorteil, falls gewünscht, jedem einzelnen Zugfaden individuell eine Spannkraft aufzuerlegen, dies mit diesen separaten Spannvorrichtungen durchgeführt werden kann.

Diese Maßnahme kann insbesondere dann ergriffen werden, wenn bei sehr großflächigen und unregelmäßig geformten Läsionen umgebende Hautbereiche vorhanden sind, die wesentlich besser einer Dehnung zugänglich sind als andere Hautbereiche. Durch die unterschiedlichen Spannvorrichtungen können dann in diesen unterschiedlichen Bereichen separate plattenförmige Körper als Gegenstück angelegt werden und durch die unterschiedliche Beaufschlagung über die Spannvorrichtung kann die Haut entsprechend unterschiedlich gedehnt werden.

In einer weiteren Ausgestaltung der Erfindung können die separaten Zugfäden von der Fadenführung einer einzigen Spannvorrichtung zugeführt werden und werden von dieser gespannt.

Diese Maßnahme hat den Vorteil, dass bei Vorhandensein mehrerer Zugfäden wie bereits zuvor erwähnt diese über eine einzige Spannvorrichtung gespannt werden können.

In einer weiteren Ausgestaltung der Erfindung sind zwei gegenüberliegende plattenförmige Körper in deren Plattenebene aufeinander zu bewegbar.

Diese Maßnahme hat insbesondere bei der Hautdehnung den Vorteil, dass die beiden plattenförmigen Körper flächig auf die Haut aufgebracht und in deren Plattenebene aufeinander zu bewegt werden. Das zwischen den sich aufeinander zu bewegenden plattenförmigen Körper vorhandene Gewebe wird gestaut, das in Bewegungsrichtung "dahinter" liegende Gewebe wird gedehnt.

In einer weiteren Ausgestaltung der Erfindung ist der zumindest erste plattenförmige Körper an einer Seite einer Läsion auf die Haut legbar und der zumindest zweite plattenförmige Körper ist an einer anderen, gegenüberliegenden Seite der Läsion auf die Haut legbar und die beiden plattenförmigen Körper sind jeweils mit der Haut verbindbar und außerdem subkutan untereinander verbindbar.

Diese Maßnahme hat den Vorteil, dass sowohl die Fixierung der plattenförmigen Körper an der Haut als auch die Verbindung der beiden plattenförmigen Körper untereinander durch einen sehr einfachen, minimal-invasiv durchzuführenden Vorgang bewerkstelligt werden können. So kann beispielsweise der Zugfaden mittels einer Ahle von dem einen Körper subkutan zum anderen plattenförmigen Körper und wieder zurückgeführt werden, um dadurch die Zugverbindung zu bewerkstelligen.

In einer weiteren Ausgestaltung der Erfindung sind die plattenförmigen Körper aus einem formbaren Material hergestellt.

Diese Maßnahme hat den Vorteil, dass bei gekrümmten Gewebebereichen die plattenförmigen Körper individuell an die entsprechende Krümmung angepasst werden können. Dies kann nun einerseits so erfolgen, dass die plattenförmigen Körper selbst aus einem verformbaren Material bestehen. Andererseits ist es auch möglich, zunächst Abdrücke von den entsprechend gekrümmten Gewebebereichen anzufertigen und anhand dieser Abdrücke dann die plattenförmigen Körper auszuformen. Soll beispielsweise ein Gefäß gedehnt werden, weil es aufgrund einer Verletzung verkürzt war, können die plattenförmigen Körper an die Krümmung des Gefäßes exakt angepasst werden.

In einer weiteren Ausgestaltung der Erfindung sind die plattenförmigen Körper entsprechend den Gewebebereichen gekrümmt.

Diese Maßnahme hat den Vorteil, dass die plattenförmigen Körper entsprechend bündig auf dem Gewebe aufliegen und somit die Zugkraft gleichmäßig verteilt auf das Gewebe ausgeübt wird.

In einer weiteren Ausgestaltung der Erfindung ist die Spannvorrichtung drehbar, wobei zumindest ein Zugfaden aufwickelbar ist.

Diese an sich aus der eingangs erwähnten Druckschrift bekannte Ausgestaltung hat den Vorteil, dass mit einer kompakten Bauweise und einer einfach zu bedienenden Vorrichtung der Spannvorgang durchführbar ist und der eingezogene Faden gleich platzsparend verstaut wird.

In einer weiteren Ausgestaltung der Erfindung weist die Spannvorrichtung einen Rastmechanismus auf, der ein Drehen der Spannvorrichtung entgegen einer Zugfadenspannkraft sperrt.

Diese ebenfalls an sich bekannte Maßnahme hat den Vorteil, dass der Patient selbständig die Spannvorrichtung um eine bestimmte Rastnasen- bzw. Zähnezahl in Spannrichtung verdreht, ein Rückdrehen aber durch den Rastmechanismus gesperrt ist.

Zum Einrichten der Vorrichtung, sprich also zum Anlegen des Fadens bzw. später zum Lösen des Fadens kann dann eine entsprechende Mechanik vorgesehen sein, um diesen Rastmechanismus wieder aufzuheben.

In einer weiteren Ausgestaltung der Erfindung steht die Spannvorrichtung von einer Seite des plattenförmigen Körpers vor und ist von Hand bedienbar.

Diese Maßnahme hat den handhabungsmäßigen Vorteil, dass die Spannvorrichtung die Anlagestellen der plattenförmigen Körper am Gewebe nicht beeinträchtigt und die Spannvorrichtung für die Manipulation zum Spannen des Fadens einfach zugänglich ist. Der Begriff "von Hand bedienbar" bedeutet auch, dass der Patient zum Drehen ein Werkzeug zu Hilfe nehmen kann, er aber selbst das Spannen des Zugfadens steuert.

Es versteht sich, dass die vorstehend genannten und die nachstehend noch zu erläuternden Merkmale nicht nur in den jeweils angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand einiger ausgewählter Ausführungsbeispiele in Zusammenhang mit den beiliegenden Zeichnungen näher beschrieben und erläutert. Es zeigen:
- Fig. 1: stark schematisch eine Draufsicht auf ein erstes Ausführungsbeispiel einer erfindungsgemäßen Vorrichtung zur Dehnung der Haut, die bereits beidseits an eine Läsion an einer Haut angelegt und fixiert ist,
- Fig. 2: eine der Fig. 1 vergleichbare Draufsicht nach Beendigung des Hautdehnvorganges,
- Fig. 3: einen Schnitt längs der Linie III-III in Fig. 1,
- Fig. 4: einen Schnitt längs der Linie IV-IV in Fig. 2,
- Fig. 5: eine der Darstellung von Fig. 1 vergleichbare Darstellung eines zweiten Ausführungsbeispiels einer Vorrichtung zum Dehnen von Hautbereichen, bei der an einer Seite zwei plattenförmige Körper angelegt sind, und
- Fig. 6: in der oberen Hälfte ein drittes Ausführungsbeispiel einer Vorrichtung zum Dehnen von Hautbereichen, bei der die beiden gegenüberliegenden plattenförmigen Körper über jeweils separate Zugfäden verbunden sind, die auf eine einzige Spannvorrichtung aufgewickelt werden, und in der unteren Hälfte ein viertes Ausführungsbeispiel, bei dem jeder Faden einer diesem zugeordneten eigenen Spannvorrichtung zugeführt wird.

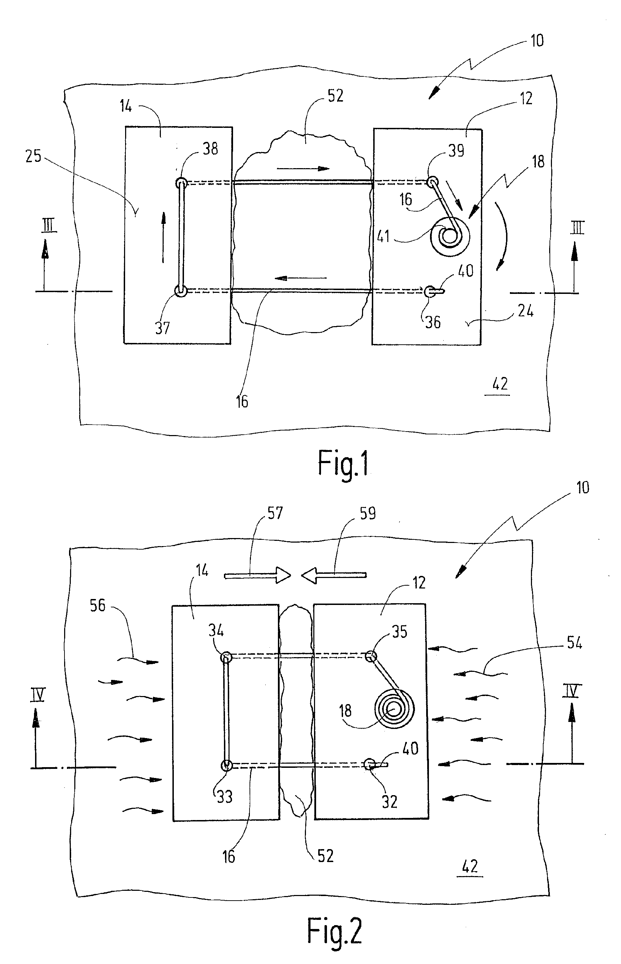

Ein in den Fig. 1 bis 4 dargestelltes erstes Ausführungsbeispiel einer erfindungsgemäßen Vorrichtung zum Dehnen von Hautbereichen beidseits einer zu exzidierenden Läsion ist in seiner Gesamtheit mit der Bezugsziffer 10 bezeichnet.

Die Vorrichtung 10 weist einen ersten, etwa rechteckförmigen plattenförmigen Körper 12 und einen etwa gleich großen zweiten ebenen plattenförmigen Körper 14 auf.

Beide plattenförmigen Körper 12 und 14 sind aus Medizinerstahl hergestellt. Es können auch Kunststoffe eingesetzt werden, wie sie in der Zahnprothetik Verwendung finden. Ein Zugfaden 16 verbindet die beiden plattenförmigen Körper 12 und 14 miteinander, wobei ein Ende 41 des Zugfadens 16 an einer auf der Oberseite 24 des ersten plattenförmigen Körpers 12 hochstehenden Spannvorrichtung 18 fixiert ist. Das andere Ende 40 ist ebenfalls an dem plattenförmigen Körper 12 fixiert.

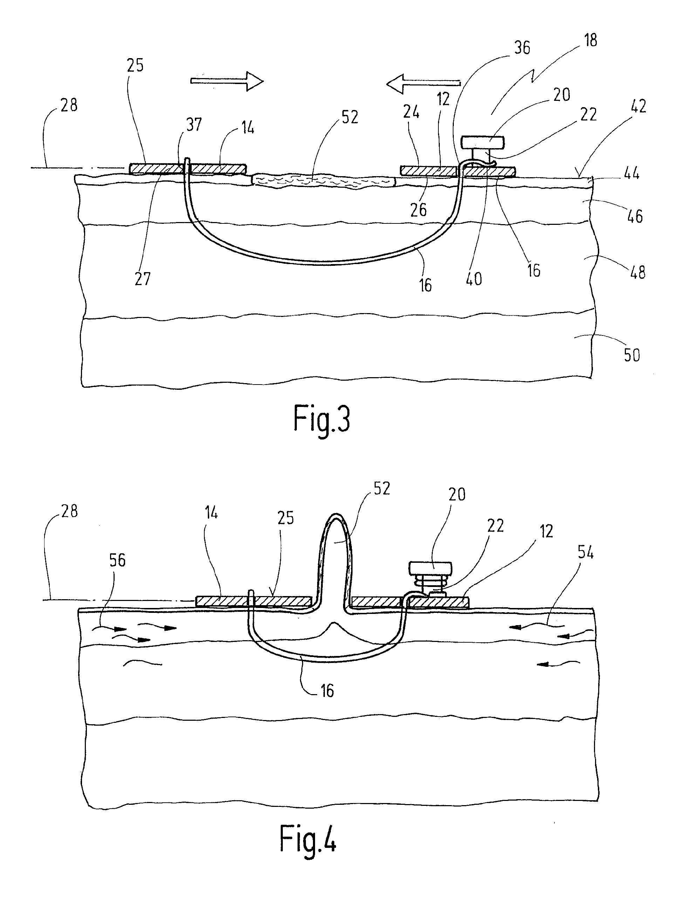

Wie insbesondere aus Fig. 3 zu erkennen, weist die Spannvorrichtung 18 einen äußeren Drehknopf 20 auf, der mit einer Nabe 22 verbunden ist. An der Nabe 22 ist das Ende 41 des Zugfadens 16 fixiert und ein Drehen des Drehkopfes 20 verursacht ein Drehen der Nabe 22 und ein Aufwickeln des Zugfadens 16 auf die Spannvorrichtung 18. Wie insbesondere aus Fig. 3 zu erkennen, liegen beide plattenförmigen Körper 12 und 14 mit ihrer jeweiligen Unterseite 26 bzw. 27 flächig auf einer Haut 42 beidseits einer Läsion 52 an, erstrecken sich also beide etwa in einer Plattenebene 28.

Wie insbesondere aus Fig. 1 zu erkennen, sind die beiden plattenförmigen Körper 12 und 14 so angelegt, dass diese etwa parallel zueinander ausgerichtet sind, so dass sich die beiden gegenüberliegenden Längskanten etwa parallel gegenüberstehen. Über die nachfolgend zu beschreibende Fadenführung sind die beiden plattenförmigen Körper 12 und 14 untereinander bzw. jeweils mit der Haut 42 verbunden.

Dazu ist am ersten plattenförmigen Körper 12, wie das insbesondere in Fig. 2 bezeichnet ist, eine erste Fixierstelle 32 vorgesehen, in deren Bereich ein erstes Ende 40 des Zugfadens 16 fixiert ist. Diese Fixierung erfolgt auf der Oberseite 24 des plattenförmigen Körpers 12. Aus Fig. 1 und 3 ist zu erkennen, dass im Bereich dieser Fixierstelle 32 eine durch den ersten plattenförmigen Körper 12 hindurchgehende runde Öffnung 36 vorgesehen ist.

Durch diese Öffnung 36, die abgerundete Kanten aufweist, wird der Zugfaden 16 von der Fixierstelle 32 von der Oberseite 24 her durch den plattenförmigen Körper 12 hindurchgeführt.

Die Fixierung des Fadenendes 40 kann entweder so erfolgen, dass dieses über eine aufzubringende Niet oder dergleichen befestigt wird, oder dass dieses Fadenende 40 schlicht und einfach mehrfach verknotet wird, so dass der Knoten dann nicht durch die Öffnung 36 hindurchtreten kann.

An dem dem ersten plattenförmigen Körper 12 gegenüberliegenden zweiten plattenförmigen Körper 14 ist, wie insbesondere aus Fig. 1 und 3 zu erkennen, etwa an gleicher Lage eine entsprechende Öffnung 37 vorgesehen, durch die der Zugfaden 16 von der Unterseite 27 her durch den plattenförmigen Körper 14 hindurchgeführt werden kann. Der Zugfaden 16 wird von der Öffnung 36 im ersten plattenförmigen Körper 12 zur Öffnung 37 im zweiten plattenförmigen Körper 14 subkutan geführt, wie das insbesondere aus der Schnittdarstellung von Fig. 3 ersichtlich ist, d.h. der Zugfaden 16 wird durch die Oberhaut 44, die Unterhaut 46 bis in das Unterhautfettgewebe 48 eingestochen und unter der Läsion 52 hindurch zur Unterseite 27 des zweiten plattenförmigen Körpers 14 geführt.

Wie insbesondere aus Fig. 1 zu erkennen, ist in dem zweiten plattenförmigen Körper 14 noch eine zweite Öffnung 38 vorhanden, durch die der Zugfaden 16 wieder von der Oberseite 25 durch den zweiten plattenförmigen Körper 14 hindurchgeführt werden kann. Auch dieser Fadenabschnitt wird dann wie zuvor beschrieben subkutan zum ersten plattenförmigen Körper 12 zurückgeführt und tritt dort durch eine Öffnung 39 von der Unterseite 26 durch den ersten plattenförmigen Körper 12 hindurch und wird dann an seinem zweiten Ende 41 auf die Nabe 22 aufgefädelt. Die Öffnung 39 wirkt somit als eine Führungsstelle für den Aus- oder Durchtritt des Zugfadens 16 durch den ersten plattenförmigen Körper 12.

Aus der Draufsicht von Fig. 1 ist zu erkennen, dass bei dieser Anordnung von erstem und zweitem plattenförmigem Körper 12 und 14 die vier Öffnungen 36, 37, 38, 39 an den Ecken eines Rechteckes liegen.

Die beiden Öffnungen 37 und 38 in dem zweiten plattenförmigen Körper 14 wirken als Widerlager 33 und 34, wenn auf den Zugfaden 16 über die Spannvorrichtung 18 eine Zugkraft ausgeübt wird.

Die plattenförmigen Körper können noch mehrere solche Öffnungen aufweisen, somit als Lochplatten hergestellt sein, so dass je nach den Gegebenheiten unterschiedliche Öffnungen für die Fadenführung herangezogen werden können. Somit kann ein plattenförmiger Körper mit einem vielzahligen Lochmuster für unterschiedlich große oder ausgeformte Läsionen herangezogen werden.

Dies erfolgt dann, wenn bei dem in Fig. 1 dargestellten Zusammenbau der Vorrichtung 10 der Drehknopf 20 im Uhrzeigersinn bewegt wird, wie das durch einen Pfeil angedeutet ist. Da beide Fadenenden 41 und 40 auf dem ersten plattenförmigen Körper 12 fixiert sind, verursacht die Zugkraft, dass die beiden plattenförmigen Körper 12 und 14 aufeinander zu bewegt werden, so dass ein Zustand erreicht wird, wie er in Fig. 2 dargestellt ist.

Das heißt, beide plattenförmigen Körper 12 und 14 haben sich aufeinander zu bewegt, wie das durch die Pfeile 57 und 59 in Fig. 2 angedeutet ist.

Die in der jeweiligen Bewegungsrichtung (Pfeile 57 und 59) der plattenförmigen Körper 12 und 14 "dahinter" gelegenen Hautbereiche 54 und 56 werden dabei gedehnt, was der wesentliche Zweck der Vorrichtung 10 ist.

Zugleich wird, wie das insbesondere aus Fig. 2 und 4 ersichtlich ist, der zwischen den aufeinander zu laufenden Kanten der plattenförmigen Körper 12 und 14 gelegene Bereich der Haut, nämlich der Bereich der Läsion 52, gestaucht bzw. gequetscht. Bei der Exzision kann dann dieser Bereich einfach ergriffen und abgetrennt werden.

Der Übergang von Fig. 1 zu Fig. 2 kann sukzessive innerhalb weniger Tage erreicht werden, indem nach und nach der Drehknopf 20 immer weiter im Uhrzeigersinn bewegt wird.

Bezüglich der näheren konstruktiven Ausgestaltung der Spannvorrichtung 18 wird ausdrücklich auf die Spannvorrichtung wie sie aus der DE 103 49 953 A1 beschrieben ist, Bezug genommen, d.h. die dort beschriebene Konstruktion wird explizit als Offenbarung in die vorliegende Anmeldung übernommen. Somit ist auch ein hier nicht näher dargestellter Rastmechanismus vorhanden, der verhindert, dass sich der Drehknopf 20 aufgrund der Spannkraft entgegen dem Uhrzeigersinn dreht.

Nachdem einem Patienten die beiden plattenförmigen Körper 12 und 14 in der in Fig. 1 dargestellten Weise platziert wurden und durch den Zugfaden 16 sowohl an der Haut 42 als auch untereinander verbunden worden sind, wird vom Arzt ein bestimmtes Regime vorgegeben, wie viel und wie oft er in den nächsten Tagen den Drehknopf 20 bewegen soll, bis der in Fig. 2 dargestellte Zustand erreicht wird.

Da dazu keine weiteren Eingriffe im Körper notwendig sind und der Drehknopf 20 vom Patienten einfach zugänglich ist, kann er das selbst ohne ärztliche Unterstützung durchführen.

Während der Exzision der Läsion werden dann die plattenförmigen Körper 12 und 14 wieder abgenommen, wozu der Zugfaden 16 aufgetrennt und verworfen wird. Nach einer Reinigung und Sterilisierung ist die Vorrichtung 10 für einen weiteren Einsatz bereit.

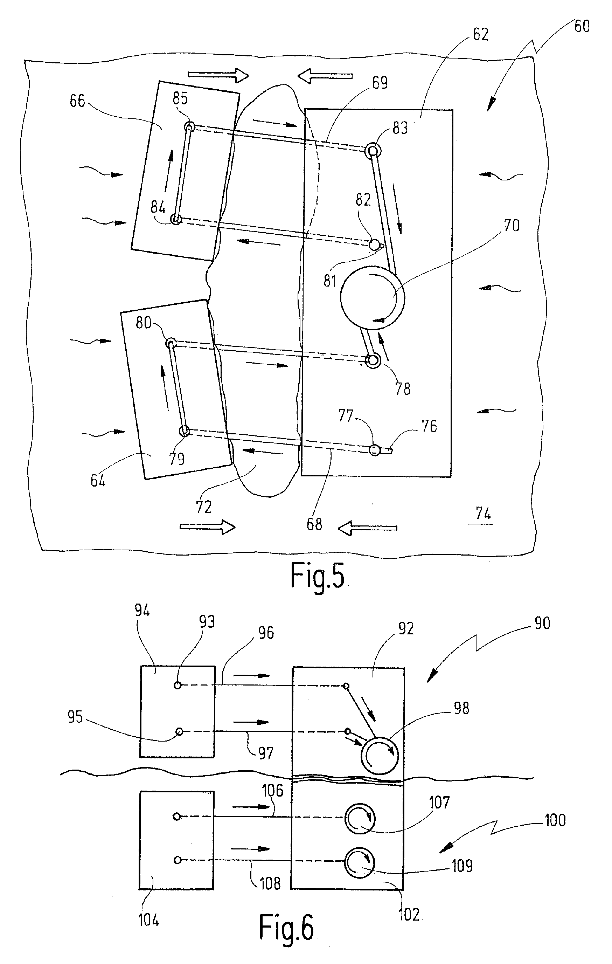

In Fig. 5 ist ein zweites Ausführungsbeispiel einer erfindungsgemäßen Vorrichtung gezeigt, die in ihrer Gesamtheit mit der Bezugsziffer 60 bezeichnet ist.

Auch diese Vorrichtung 60 arbeitet prinzipiell gleich wie das zuvor beschriebene erste Ausführungsbeispiel und wird dementsprechend an der Haut 74 eines Patienten fixiert.

Im Gegensatz zu der zuvor beschriebenen Vorrichtung 10 ist bei der Vorrichtung 60 ein relativ großer erster plattenförmiger Körper 62 vorhanden, der ebenfalls rechteckförmig ist und an einer Seite einer relativ langen Läsion 72 angelegt wird.

Im Gegensatz zum zuvor beschriebenen Ausführungsbeispiel sind auf der dem ersten plattenförmigen Körper 62 gegenüberliegenden Seite der Läsion zwei plattenförmige Körper 64 und 66 angelegt, die etwa nur halb so groß wie der erste plattenförmige Körper 62 sind. Aus Fig. 5 ist zu erkennen, dass die Läsion eine gekrümmte Kontur hat, so dass die beiden Kanten des zweiten und dritten plattenförmigen Körpers 64 und 66, die der an der Läsion 72 anliegenden Kante des ersten plattenförmigen Körpers 62 gegenüber liegen, somit schräg zu dieser Kante verlaufen.

Der zweite plattenförmige Körper 64 ist wie zuvor beschrieben über einen Zugfaden 68 mit dem ersten plattenförmigen Körper 62 verbunden. Der dritte plattenförmige Körper 66 ist über einen weiteren Zugfaden 69 mit dem ersten plattenförmigen Körper 62 verbunden.

Auch hier ist dann die Fadenführung wieder so, dass an einer Fixierstelle 76 ein erstes Ende des Zugfadens 68 fixiert ist, dieser durch eine erste Öffnung 77 durch den ersten plattenförmigen Körper 62 hindurch und subkutan dann zu einer ersten Öffnung 79 im zweiten plattenförmigen Körper 64 geführt ist. Dieser Zugfaden 68 wird dann wie zuvor beschrieben über die Oberseite des zweiten plattenförmigen Körpers 64 zu der zweiten Öffnung 80 geführt und dann wieder durch die Platte hindurch und subkutan zu der Öffnung 78 im ersten plattenförmigen Körper 62 geführt. Von dieser Öffnung wird dann das Fadenende zu einer Spannvorrichtung 70 geführt, dort fixiert und aufgefädelt.

Entsprechend wird der zweite Zugfaden 69 von einer Fixierstelle 81 über eine Öffnung 82 in dem ersten plattenförmigen Körper 62 subkutan zu einer ersten Öffnung 84 im dritten plattenförmigen Körper 66 geführt, dort über dessen Oberfläche zu der zweiten Öffnung 85 im dritten plattenförmigen Körper 66 geführt und durch diesen hindurchgeführt und anschließend subkutan zu einer vierten Öffnung 83 im ersten plattenförmigen Körper 62 geführt. Aus dieser Öffnung 83 tritt der zweite Zugfaden 69 aus und wird ebenfalls der Spannvorrichtung 70 zugeführt.

Durch Drehen der Spannvorrichtung 70 im Uhrzeigersinn, wie das durch einen Pfeil angedeutet ist, werden beide Zugfäden 68 und 69 gespannt bzw. aufgewickelt, so dass auf beide plattenförmigen Körper 64 und 66 eine Zugkraft ausgeübt wird. Auch hier dienen wieder die Öffnungen 79 und 80 bzw. 84 und 85 in den plattenförmigen Körpern 64 und 66 als entsprechende Widerlager. Auch hier erfolgt ein Aufeinanderzubewegen von dem erstem plattenförmigen Körper 62 einerseits und den beiden plattenförmigen Körpern 64 und 66 andererseits, wodurch dann die gewünschte Hautdehnung erzielt wird.

Das Funktionsprinzip ist dasselbe wie zuvor beschrieben, es werden hier eben nur aufgrund der Geometrie der Läsion mehrere Gegenkörper in Form der plattenförmigen Körper 64 und 66 eingesetzt.

Es ist einleuchtend, dass bei entsprechend längeren oder anderen Ausformungen einer Läsion 72 auch mehr als zwei oder geometrisch anders geformte plattenförmige Körper 64 und 66 eingesetzt werden können.

Es ist auch möglich, an die relativ lang verlaufende Läsion 72 von Fig. 5 zwei Vorrichtungen 10, wie sie zuvor beschrieben sind, anzulegen.

Es ist zu sehen, dass hier sehr viele konstruktive Ausgestaltungen möglich sind, um möglichst individuell auf die Beschaffenheit der Haut und der Läsion anpassen zu können, wobei immer dasselbe Grundprinzip zugrunde liegt.

Bei den in Fig. 6 dargestellten weiteren Ausführungsbeispielen ist auf der oberen Hälfte eine Vorrichtung 90 dargestellt, die im Prinzip zunächst einmal so aufgebaut ist, wie das Ausführungsbeispiel von Fig. 1, d.h. es ist ein erster plattenförmiger Körper 92 vorhanden, dem ein zweiter plattenförmiger Körper 94 gegenüberliegt. Im Gegensatz zu dem ersten Ausführungsbeispiel sind hier beim dritten Ausführungsbeispiel zwei separate Zugfäden 96 und 97 vorhanden, die jeweils auf ein und dieselbe Spannvorrichtung 98 auf den ersten plattenförmigen Körper 92 zugeführt werden. Die jeweils anderen Enden der Zugfäden 96 und 97 sind an den entsprechenden Öffnungen 93 und 95 im zweiten plattenförmigen Körper 94 fixiert, beispielsweise durch einen Knoten.

Bei der in Fig. 1 dargestellten ersten Ausgestaltung mit nur einem einzigen Zugfaden läuft der Zugfaden 16 beim Spannen über die Oberseite 25 des zweiten plattenförmigen Körpers 14. Das heißt, ein solcher Abschnitt des Zugfadens 16 tritt über die Öffnung 38 durch den zweiten plattenförmigen Körper 14 hindurch und in das subkutane Gewebe hinein.

Daher müssen entsprechende Maßnahmen vorgesehen sein, dass dieser Fadenabschnitt zwischen den Widerlagern bzw. Öffnungen 37 und 38 nicht kontaminiert und gegebenenfalls eine Infektion verursachen kann. Es müssen entsprechende Desinfektions- oder Abdeckmaßnahmen oder im zweiten plattenförmigen Körper 14 ein innerer Kanal vorgesehen sein, in dem der Fadenabschnitt steril geführt ist, um ein solches Infektionsrisiko zu minimieren.

Bei der in Fig. 6 auf der oberen Hälfte gezeigten Konstruktion entfällt dieses, da hier zwei separate Zugfäden 96 und 97 vorhanden sind, deren jeweils eines Ende an den Öffnungen 93 und 95 fixiert sind.

In Fig. 6 ist an der unteren Hälfte ein viertes Ausführungsbeispiel einer erfindungsgemäßen Vorrichtung 100 dargestellt, die im Prinzip gleich wie die Vorrichtung 90 und auch die zuvor beschriebenen Vorrichtungen aufgebaut und an der Haut befestigt wird, mit dem Unterschied, dass die beiden einzelnen Zugfäden 106, 108 jeweils einer einzelnen Spannvorrichtung 107, 109 auf dem ersten plattenförmigen Körper 102 zugeführt werden. Dadurch ist es möglich, über die Spannvorrichtung 107 auf den Zugfaden 106 eine andere Zugkraft auszuüben als mit der Spannvorrichtung 109 auf den Zugfaden 108, mit der Folge, dass der zweite plattenförmige Körper 104 beispielsweise im Laufe der Bewegung gekippt oder schräg gestellt werden kann, wenn das aus bestimmten Gründen sinnvoll oder notwendig sein sollte, um die gewünschte Hautdehnung zu erzielen.

Zusammenfassend ist zu sagen, dass zahlreiche Ausgestaltungen der erfindungsgemäßen Vorrichtung möglich sind, um individuell an die örtlichen Gegebenheiten im Bereich der Haut und der Läsion anzupassen, alle aber einem gleichen Grundprinzip unterliegen.

Dieses Grundprinzip ist der einfache Aufbau durch die plattenförmigen Körper und das einfache Fixieren dieser Körper an der Haut und Verbinden untereinander über die subkutane Fadenführung.

## Patentansprüche

1. Vorrichtung zum Dehnen von Gewebebereichen (54, 56), mit zumindest einem ersten plattenförmigen Körper (12, 62, 92, 102), und mit zumindest einem zweiten plattenförmigen Körper (14, 64, 94, 104), die gegenüberliegend am Gewebe anordenbar sind, mit zumindest einem Zugfaden (16; 68, 69; 96, 97; 106, 108), der die beiden plattenförmigen Körper (12, 62, 92, 102; 14, 64, 94, 104) untereinander verbindet, ferner mit zumindest einer Spannvorrichtung (18, 70, 98, 107) auf zumindest einem der beiden plattenförmigen Körper (12, 62, 92, 102; 14, 64, 94, 104) zum Beaufschlagen des zumindest einen Zugfadens (16; 68, 69; 96, 97; 106, 108) mit einer Zugkraft, wodurch die beiden plattenförmigen Körper (12, 62, 92, 102; 14, 64, 94, 104) aufeinander zu bewegbar sind, und mit einer Fadenführung, über die die die beiden plattenförmigen Körper (12, 62, 92, 102; 14, 64, 94, 104) zugkraftschlüssig untereinander verbindbar sind, **dadurch gekennzeichnet, dass** die Fadenführung auf dem plattenförmigen Körper, der dem mit einer Spannvorrichtung gegenüberliegt, zwei seitlich voneinander beabstandete Widerlager (33, 34) aufweist.

2. Vorrichtung nach Anspruch 1, **dadurch gekennzeichnet, dass** die Fadenführung an dem plattenförmigen Körper (12), der eine Spannvorrichtung (18) trägt, zumindest eine Führungsstelle (35) aufweist, über die der Zugfaden (16) der Spannvorrichtung (18) zuführbar ist und dort an einem ersten Ende (41) aufwickelbar ist.

3. Vorrichtung nach Anspruch 1 oder 2, **dadurch gekennzeichnet, dass** die Fadenführung an dem plattenförmigen Körper (12), der eine Spannvorrichtung (18) trägt, eine Fixierstelle (32) für ein zweites Ende (40) des Zugfadens (16) aufweist.

4. Vorrichtung nach einem der Ansprüche 1 bis 3, **dadurch gekennzeichnet, dass** die Widerlager (33, 34) auf dem einen plattenförmigen Körper (14) und eine Führungsstelle (35) und eine Fixierstelle (32) am anderen plattenförmigen Körper (12) an den Ecken eines Viereckes liegen.

5. Vorrichtung nach einem der Ansprüche 1 bis 4, **dadurch gekennzeichnet, dass** die Widerlager (33 und 34) und die Fixierstelle (32) und die Führungsstelle (35) als Öffnungen (36 bis 39) in den plattenförmigen Körper (12, 14) ausgebildet sind, über die der zumindest eine Zugfaden (16) durch die Plattenebene (28) hindurchführbar ist.

6. Vorrichtung nach einem der Ansprüche 1 bis 5, **dadurch gekennzeichnet, dass** auf einer Seite mehr als ein plattenförmiger Körper (64, 66) vorhanden ist, die jeweils mit einem gegenüberliegend angeordneten plattenförmigen Körper (62) über jeweils einen Zugfaden (68, 69) verbunden sind.

7. Vorrichtung nach Anspruch 6, **dadurch gekennzeichnet, dass** ein plattenförmiger Körper (62) eine Spannvorrichtung (70) aufweist, und dass dieser plattenförmige Körper (62) mit mehreren plattenförmigen Körpern (64, 66) auf einer gegenüberliegenden Seite über jeweils einen Zugfaden (68, 69) in Verbindung steht und dass die Zugfäden (68, 69) der mehreren plattenförmigen Körper (64, 66) zu einer einzigen Spannvorrichtung (70) durch die Fadenführung geführt sind und von dieser einzigen Spannvorrichtung (70) gleichzeitig spannbar sind.

8. Vorrichtung nach einem der Ansprüche 1 bis 7, **dadurch gekennzeichnet, dass** zwei gegenüberliegende plattenförmige Körper (92, 94; 102, 104) über jeweils zwei separate Zugfäden (96, 97; 106, 108) untereinander verbunden sind.

9. Vorrichtung nach Anspruch 8, **dadurch gekennzeichnet, dass** die separaten Zugfäden (96, 97) von der Fadenführung einer einzigen Spannvorrichtung (98) zugeführt werden und von dieser spannbar ist.

10. Vorrichtung nach Anspruch 8, **dadurch gekennzeichnet, dass** jedem separaten Zugfaden (106, 108) eine separate Spannvorrichtung (107, 109) zugeordnet ist.

11. Vorrichtung nach einem der Ansprüche 1 bis 10, **dadurch gekennzeichnet, dass** zwei gegenüberliegende plattenförmige Körper (12, 62, 92, 102; 14, 64, 94, 104) in deren Plattenebene (28) aufeinander zu bewegbar sind.

12. Vorrichtung nach einem der Ansprüche 1 bis 11, **dadurch gekennzeichnet, dass** der zumindest eine erste plattenförmige Körper (12, 62, 92, 102) an einer Seite eine Läsion (52, 57) auf die Haut (42, 74) legbar ist, und der zumindest zweite plattenförmige Körper (14, 64, 94, 104) an einer anderen, gegenüberliegenden Seite der Läsion (52, 72) auf die Haut (42, 72) legbar ist und dass die beiden plattenförmigen Körper (12, 62, 92, 102; 14, 64, 94, 104) jeweils mit der Haut verbindbar sind und außerdem subkutan untereinander verbindbar sind.

13. Vorrichtung nach einem der Ansprüche 1 bis 12, **dadurch gekennzeichnet, dass** die plattenförmigen Körper eine gekrümmte Plattenebene aufweist, die einer Krümmung des Gewebes angepasst ist.

14. Vorrichtung nach einem der Ansprüche 1 bis 13, **dadurch gekennzeichnet, dass** die plattenförmigen Körper aus einem formbaren Material bestehen.

15. Vorrichtung nach einem der Ansprüche 1 bis 14, **dadurch gekennzeichnet, dass** die Spannvorrichtung (18, 70, 98, 107, 109) drehbar ist, wobei zumindest ein Zugfaden aufwickelbar ist.

16. Vorrichtung nach einem der Ansprüche 1 bis 15, **dadurch gekennzeichnet, dass** die Spannvorrichtung einen Rastmechanismus aufweist, der ein Drehen der Spannvorrichtung entgegen einer Zugfadenspannkraft sperrt.

17. Vorrichtung nach einem der Ansprüche 1 bis 16, **dadurch gekennzeichnet, dass** die Spannvorrichtung von einer Seite eines plattenförmigen Körpers vorsteht.

## Claims

1. Device for stretching regions of tissue (54, 56), having at least one first plate-like body (12, 62, 92, 102) and at least one second plate-like body (14, 64, 94, 104), which can be arranged lying opposite on the tissue, at least one pulling thread (16; 68, 69; 96, 97; 106, 108), which joins the two plate-like bodies (12, 62, 92, 102; 14, 64, 94, 104) to one another, further having at least one tensioning device (18, 70, 98, 107) on at least one of the two plate-like bodies (12, 62, 92, 102; 14, 64, 94, 104), for applying a tensile force to the at least one pulling thread (16; 68, 69; 96, 97; 106, 108), whereby the plate-like bodies (12, 62, 92, 102; 14, 64, 94, 104) can be moved towards one another, and a thread guidance, by means of which the two plate-like bodies (12, 62, 92, 102; 14, 64, 94, 104) can be joined together in such a way that they are fixed in terms of tensile force, **characterized in that** the thread guidance on that plate-like body that lies opposite to the one with tensioning device has two abutments (33, 34) spaced laterally apart from one another.

2. Device of claim 1, **characterized in that** the thread guidance on the plate-like body (12) that carries a tensioning device (18) has at least one guiding location (35) by means of which the pulling thread (16) can be fed to the tensioning device (18) and can be wound up there at a first end (41).

3. Device of claims 1 or 2, **characterized in that** the thread guidance on the plate-like body (12), that carries a tensioning device (18) has a fixing location (32) for a second end (40) of the pulling thread (16).

4. Device of any one of claims 1 through 3, **characterized in that** the abutments (33, 34) on the one plate-like body (14) and a guiding location (35) and a fixing location (32) on the other plate-like body (12) lie at the corners of a quadrifateral.

5. Device of any one of claims 1 through 4, **characterized in that** the abutments (33 and 34) and the fixing location (32) and the guiding location (35) are formed as openings (36 to 39) in the plate-like bodies (12, 14,) by way of which the at least one pulling thread (16) can be guided through the plane of the plates (28).

6. Device of any one of claims 1 through 5, **characterized in that** on one side there is more than one plate-like body (64, 66), respectively joined to a plate-like body (62) arranged lying opposite by means of a pulling thread (68, 69) in each case.

7. Device of claim 6, **characterized in that** one plate-like body (62) has a tensioning device (70), and **in that** this plate-like body (62) is joined to a number of plate-like bodies (64, 66) on an opposite side by means of a pulling thread (68, 69) in each case, and **in that** the pulling threads (68, 69) of the number of plate-like bodies (64, 66) are guided by the thread guidance to a single tertsioning device (70) and can be tensiorted simultaneously by this single tensioning device (70).

8. Device of any one of claims 1 through 7, **characterized in that** two oppositely lying plate-like bodies (92, 94; 102, 104) are joined together by means of two separate pulling threads (96, 97; 106, 108) in each case.

9. Device of claim 8, **characterized in that** the separate pulling threads (96, 97) are fed by the thread guidance to a single tensioning device (98) and can be tensioned by it.

10. Device of claim 8, **characterized in that** each separate tensioning thread (106, 108) is assigned a separate tensioning device (107, 109).

11. Device of any one of claims 1 through 10, **characterized in that** two oppositely lying plate-like bodies (12, 62, 92, 102; 14, 64, 94, 104) can be moved towards one another in the plane of their plates (28).

12. Device of any one of claims 1 through 11, **characterized in that** the at least one first plate-like body (12, 62, 92, 102) can be placed onto the skin (42, 74) on one side of a lesion (52, 57), and the at least one second plate-like body (14, 64, 94, 104) can be placed onto the skin (42, 72) on another, opposite side of the lesion (52, 72), and **in that** the two plate-like bodies (12, 62, 92, 102; 14, 64, 94, 104) can be respectively joined to the skin and also joined together subcutaneously.

13. Device of any one of claims 1 through 12, **characterized in that** the plate-like bodies have a curved plane of the plates, which is adapted to a curvature of the tissue.

14. Device of any one of claims 1 through 13, **characterized in that** the plate-like bodies consist of a fordable material.

15. Device of any one of claim 1 through 14, **characterized in that** the tensioning device (18, 70, 98, 107, 109) is rotatable, whereby it is possible for at least one pulling thread to be wound up.

16. Device of any one of claims 1 through 15, **characterized in that** the tensioning device has a latching mechanism which blocks turning of the tensioning device counter to a pulling thread tensioning force.

17. Device of any one of claims 1 through 16, **characterized in that** the tensioning device protrudes from one side of a plate-like body.

## Revendications

1. Dispositif pour étirer des zones tissulaires (54, 56), comprenant au moins un premier corps en forme de plaque (12, 62, 92, 102) et au moins un deuxième corps en forme de plaque (14, 64, 94, 104) qui peuvent être disposés en face l'un de l'autre sur le tissu, au moins un fil de traction (16 ; 68, 69 ; 96, 97 ; 106, 108) qui relie les deux corps en forme de plaque (12, 62, 92, 102 ; 14, 64, 94, 104) entre eux, comprenant en outre au moins un dispositif tendeur (18, 70, 98, 107) sur au moins un des deux corps en forme de plaque (12, 62, 92, 102 ; 14, 64, 94, 104) pour soumettre ledit au moins un fil de traction (16 ; 68, 69 ; 96, 97 ; 106, 108) à une force de traction, de sorte que les deux corps en forme de plaque (12, 62, 92, 102 ; 14, 64, 94, 104) soient déplaçables l'un vers l'autre, et comprenant un guide-fil par lequel les deux corps en forme de plaque (12, 62, 92, 102 ; 14, 64, 94, 104) peuvent être reliés entre eux par force de traction, **caractérisé en ce que** le guide-fil sur le corps en forme de plaque qui est situé en face de celui présentant un dispositif tendeur présente deux points d'appui (33, 34) distants l'une de l'autre latéralement.

2. Dispositif selon la revendication 1, **caractérisé en ce que** le guide-fil sur le corps en forme de plaque (12) qui porte un dispositif tendeur (18) présente au moins un point de guidage (35) par lequel le fil de traction (16) du dispositif tendeur (18) peut être amené et où il peut être enroulé à une première extrémité (41).

3. Dispositif selon la revendication 1 ou 2, **caractérisé en ce que** le guide-fil sur le corps en forme de plaque (12) qui porte un dispositif tendeur (18) présente un point de fixation (32) pour une deuxième extrémité (40) du fil de traction (16).

4. Dispositif selon l'une des revendications 1 à 3, **caractérisé en ce que** les points d'appui (33, 34) sur l'un des corps en forme de plaque (14) et un point de guidage (35) et un point de fixation (32) sur l'autre corps en forme de plaque (12) sont situés aux sommets d'un quadrilatère.

5. Dispositif selon l'une des revendications 1 à 4, **caractérisé en ce que** les points d'appui (33 et 34), le point de fixation (32) et le point de guidage (35) sont réalisés sous la forme d'ouvertures (36 à 39) dans les corps en forme de plaque (12, 14), par lesquelles ledit au moins un fil de traction (16) peut être passé à travers le plan de plaque (28).

6. Dispositif selon l'une des revendications 1 à 5, **caractérisé en ce que** plus d'un corps en forme de plaque (64, 66) sont présents d'un côté, lesquels sont reliés chacun à un corps en forme de plaque (62) disposé en face d'eux par un fil de traction respectif (68, 69).

7. Dispositif selon la revendication 6, **caractérisé en ce qu'**un corps en forme de plaque (62) présente un dispositif tendeur (70), que ce corps en forme de plaque (62) est relié à plusieurs corps en forme de plaque (64, 66) sur un côté opposé par un fil de traction respectif (68, 69) et que les fils de traction (68, 69) desdits plusieurs corps en forme de plaque (64, 66) sont guidés vers un unique dispositif tendeur (70) par le guide-fil et peuvent être tendus en même temps par cet unique dispositif tendeur (70).

8. Dispositif selon l'une des revendications 1 à 7, **caractérisé en ce que** deux corps en forme de plaque (92, 94 ; 142, 104) situés l'un en face de l'autre sont reliés entre eux par chaque fois deux fils de traction séparés (96, 97 ; 106, 108).

9. Dispositif selon la revendication 8, **caractérisé en ce que** les fils de traction séparés (96, 97) sont amenés par le guide-fil à un unique dispositif tendeur (98) et peuvent être tendus par celui-ci.

10. Dispositif selon la revendication 8, **caractérisé en ce qu'**un dispositif tendeur séparé (107, 109) est associé à chaque fil de traction séparé (106, 108).

11. Dispositif selon l'une des revendications 1 à 10, **caractérisé en ce que** deux corps en forme de plaque (12, 62, 92, 102 ; 14, 64, 94, 104) situés l'un en face de l'autre sont déplaçables l'un vers l'autre dans leur plan de plaque (28).

12. Dispositif selon l'une des revendications 1 à 11, **caractérisé en ce que** ledit au moins un premier corps en forme de plaque (12, 62, 92, 102) peut être posé sur la peau (42, 74) d'un côté d'une lésion (52, 57) et ledit au moins un deuxième corps en forme de plaque (14, 64, 94, 104) peut être posé sur la peau (42, 72) d'un autre côté, opposé, de la lésion (52, 72) et que les deux corps en forme de plaque (12, 62, 92, 102 ; 14, 64, 94, 104) peuvent être reliés chacun à la peau et être reliés par ailleurs entre eux de façon sous-cutanée.

13. Dispositif selon l'une des revendications 1 à 12, **caractérisé en ce que** les corps en forme de plaque présentent un plan de plaque courbe qui est adapté à une courbure du tissu.

14. Dispositif selon l'une des revendications 1 à 13, **caractérisé en ce que** les corps en forme de plaque sont composés d'un matériau moulable.

15. Dispositif selon l'une des revendications 1 à 14, **caractérisé en ce que** le dispositif tendeur (18, 70, 98, 107, 109) est rotatif, au moins un fil de traction pouvant être enroulé.

16. Dispositif selon l'une des revendications 1 à 15, **caractérisé en ce que** le dispositif tendeur présente un mécanisme d'encliquetage qui bloque une rotation du dispositif tendeur à l'encontre d'une force de tension du fil de traction.

17. Dispositif selon l'une des revendications 1 à 16, **caractérisé en ce que** le dispositif tendeur est en saillie d'un côté d'un corps en forme de plaque.
